# EUROPÄISCHE PATENTSCHRIFT

(11) **EP 3 419 744 B1**
(45) Veröffentlichungstag und Bekanntmachung des Hinweises auf die Patenterteilung: **12.05.2021**
(21) Anmeldenummer: 17706687.5
(22) Anmeldetag: 24.02.2017
(51) Int. Cl.: B01F 17/00, C07D 295/037

(54) **ZUSAMMENSETZUNG UMFASSEND EIN FLUORHALTIGES TENSID**
COMPOSITION COMPRISING A FLUORINE-CONTAINING SURFACTANT
COMPOSITION COMPRENANT UN TENSIO-ACTIF FLUORE

(30) Priorität: 25.02.2016 EP 16157380
(43) Veröffentlichungstag der Anmeldung: 02.01.2019
(73) Patentinhaber: HWK Kronbichler GmbH, 6341 Ebbs (AT)
(72) Erfinder: HUMMEL, Michael, 6020 Innsbruck (AT); NAIER, Benjamin, 6020 Innsbruck (AT); SCHOTTENBERGER, Herwig, 6082 Patsch (AT); HUPPERTZ, Hubert, 6020 Innsbruck (AT); PARTL, Gabriel, 6521 Fliess (AT); NOISTERNING, Michael, 6465 Nassereith (AT)
(74) Vertreter: Frick, Robert
(86) Internationale Anmeldenummer: PCT/EP2017/000262
(87) Internationale Veröffentlichungsnummer: WO 2017/144181

(56) Entgegenhaltungen:
- WO-A1-2011/046796
- DE-A1- 1 966 931
- US-A- 4 242 516
- US-A1- 2015 368 182
- ] C Barsigian: "Product Data Sheet", Eye Res. J. Biol. Chem, 1. Januar 1998 (1998-01-01), Seite 531, XP055294735, Gefunden im Internet: URL:http://zedira.com/data/pds/d004.pdf [gefunden am 2016-08-09]

## Beschreibung

Die Erfindung betrifft eine Zusammensetzung umfassend ein fluorhaltiges Tensid mit einer kationischen Gruppe, einer zwei- oder mehrwertigen schwefelhaltigen Gruppe und einer fluorierten Gruppe, sowie ferner umfassend ein zu der kationischen Gruppe des fluorhaltigen Tensids korrespondierendes Anion.

Fluorhaltige Verbindungen werden in einer Reihe von Industriezweigen als Tenside verwendet. Einsatzmöglichkeiten umfassen das Beizen von Holz, die Förderung und Verarbeitung von fossilen Brennstoffen in der ÖI- und Erdgasindustrie, den Zusatz zu polymerisierbaren Zusammensetzungen, den Einsatz als nicht brennbare Tenside in Löschmitteln und insbesondere Schaum, sowie den Einsatz in Farben, Druckfarben, Reinigungsmitteln, Klebstoffen, Kontaktlinsen, Kreiden, Geweben, Textilien, Polituren und Wachsen, auf harten Oberflächen, in der Elektronik und in Metall-Beschichtungen, als Kupplungsmittel oder als Schmiermittel. Wichtige Aufgaben umfassen dabei die Verbesserung des Verlaufs, die Wirkung als Emulgator, die Verringerung der *"Orangenhauteffekts"* und der Kraterbildung, die Verbesserung des Glanzes, die Erhöhung der Sauerstofflöslichkeit, der Topf-Zeit, die Einstellung oleophober und hydrophober (amphiphober) Eigenschaften, der Kontrolle über die Schaumbildung, der Oberflächenbenetzung, der UV-Stabilität, der Wetterbeständigkeit oder die Steuerung des Quellverhaltens von Hydrogelen.

Die US 4,062,849 A beschreibt die Anwendung kationischer Fluorverbindungen als Tensid. Darin sind Verbindungen der nachfolgend wiedergegebenen Formel beschrieben.

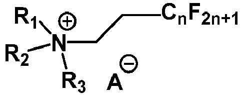

Auch die WO 2011/046796 A1 und die EP 0 256 980 A2 offenbaren kationische Fluortenside, unter anderem solche gemäß der folgenden Formeln.

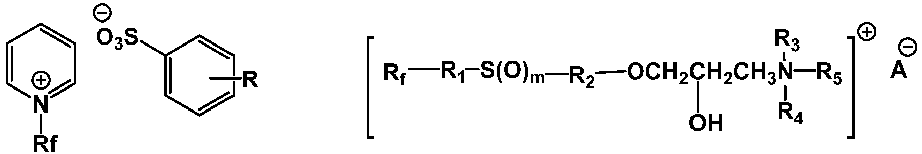

Die US 3,513,172 A und die US 8,901,311 B2 offenbaren ungeladene fluorhaltige Verbindungen der nachfolgend wiedergegebenen Struktur ohne Bezugnahme auf deren mögliche Anwendung als Tensid.

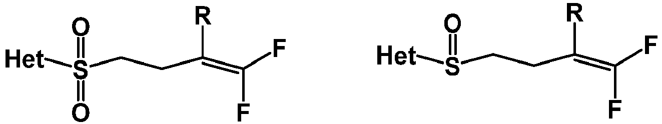

In der Anmeldung US 2006/0173190 A1 wird die Verwendung dieser Verbindungen zur Schädlingsbekämpfung angedacht.

In Mizuta, S., et al., RSC Adv., 2016, 6, 43159-43162 wird die Herstellung von N,N-Dialkyltrifluormethylthioimidazoliumsalzen mit einer oberflächenaktiven Wirkung offenbart, wobei die Herstellung durch Umsetzung eines Imidazolthions mit einem Trifluormethylreagenz erfolgt.

Die US 4,242,516 A offenbart fluorierte Mercaptane, die an Chinolinium, Piperazinium oder Pyridinium gebunden sind. Die Herstellung erfolgt durch Reaktion der ungeladenen heterocyclischen Verbindung mit dem fluorierten Mercaptan in Gegenwart von Maleinsäureanhydrid bzw. Bernsteinsäure. Die Verbindungen sind oberflächenaktiv.

Die US 2015/368182 A1 offenbart N,N-Dialkylimidazolinium- und N,N-Dialkylpyrrolidiniumfluorosulfonate, wobei diese Verbindungen aufgrund der Sulfonatgruppe Tensideigenschaften besitzen.

Ziel der vorliegenden Erfindung ist es, eine Zusammensetzung mit fluorhaltigen Tensiden bereitzustellen, die gegenüber bekannten fluorhaltigen Tensiden verbesserte Eigenschaften aufweisen und synthetisch einfach zugänglich sind. Auch die Abbaubarkeit und die Umweltverträglichkeit soll in bevorzugten Ausführungsformen verbessert werden.

Vor diesem Hintergrund betrifft die Erfindung eine Zusammensetzung umfassend ein fluorhaltiges Tensid mit einer kationischen Gruppe, einer zwei- oder mehrwertigen schwefelhaltigen Gruppe und einer fluorierten Gruppe, sowie ferner umfassend ein zu der kationischen Gruppe des fluorhaltigen Tensids korrespondierendes Anion, wobei es sich bei der kationischen Gruppe um eine N,N-disubstituierte Imidazoliumgruppe, eine N,N-disubstituierte Benzimidazoliumgruppe oder eine N,N-disubstituierte Benzimidazoliniumgruppe handelt.

Gegenüber den beispielsweise aus der US 4,062,849 A und der EP 0 256 980 A2 vorbekannten Zusammensetzungen lassen sich die erfindungsgemäßen Zusammensetzungen wesentlich kostengünstiger herstellen und die Struktur und damit Eigenschaften der Tenside können synthetisch in einfacher Weise verändert werden.

Die fluorierte Gruppe enthält C-F Bindungen. Von der fluorierten Gruppe geht eine hydrophobe und oleophobe (amphiphobe) Wirkung aus. Die kationische Gruppe ermöglicht die Lösung der Fluortenside in Lösungsmitteln mit höherer Polarität und erhöht die Wechselwirkung mit Substraten, die zumindest eine negative Partialladung aufweisen. Zusätzlich können durch die kationische Ladung der Fluortenside auch spezifische Anionen eingebracht werden, welche ihrerseits einen Effekt auf die Oberflächenaktivität der Zusammensetzung haben.

In einer Ausführungsform umfassen die Tenside genau eine kationische Gruppe.

Bei der fluorierten Gruppe handelt es sich erfindungsgemäß um eine vollständig fluorierte und gegebenenfalls lineare Gruppe vom Typ -(CF₂)ₙ-CF₃, wobei n zwischen 2 und 9 liegt und vorzugsweise 4, 5 oder 6 ist. Als vollständig fluorierte Kohlenwasserstoffgruppe ist eine Perfluoroorganylgruppe zu verstehen, in der alle Wasserstoffatome durch Fluoratome ersetzt sind. Eine gesamte Kettenlänge von 5 bis 7 fluorierten C-Atomen ist besonders vorteilhaft, da die hydrophoben und oleophoben Eigenschaften der Fluortenside bei dieser Kettenlänge bereits sehr stark ausgeprägt sind, gleichzeitig aber noch eine gute Umweltverträglichkeit gegeben ist. Besonders bevorzugt ist der Einsatz einer Perfluorhexylgruppe mit der Strukturformel -(CF₂)₅-CF₃.

In einer Ausführungsform kann die fluorierte Kette aus einem Fluorether bestehen. Eine Synthese derartiger Ether ist in Skalicky et al., Organometallics, 2012, 31 (4), pp 1524-1532 beschrieben.

In einer Ausführungsform umfassen die Tenside genau eine fluorierte Kette.

In einer Ausführungsform handelt es sich bei der schwefelhaltigen Gruppe um einen Thioether bzw. eine Sulfidbrücke, sodass im Tensid ein an die kationische Gruppe substituierter Thioether (eine Organylsulfidgruppe) vorliegt. Alternative schwefelhaltige Gruppen umfassen Polysulfide, Sulfide, Disulfide, Sulfoxide und Sulfone. Ein derartiger Thioether kann durch Substitutionsreaktion aus einer Thiongruppe und beispielsweise einem halogenierten Kohlenwasserstoff erhalten werden. Zusätzlich kann diese durch gezielte Oxidation in eine Sulfoxid- und eine Sulfonatgruppe überführt werden. Des Weiteren kann auch ein Thion oxidativ in ein Disulfid übergeführt werden, welches danach ebenfalls positiv geladen ist.

In einer Ausführungsform umfassen die Fluortenside genau eine schwefelhaltige Gruppe.

In einer Ausführungsform liegt das Anion separat in der Zusammensetzung vor, ist also nicht kovalent an das Tensid gebunden. Geeignete Anionen umfassen beispielsweise Fluorid, Chlorid, Bromid, lodid, Arylsulfonat, Alkylsulfonat, Alkylsulfat, Sulfat, Arylphosphonat, Alkylphosphonat, Monoalkylphosphat, Tetrafluoroborat, Dialkylphosphat, (Di)Hydrogenphosphat, Phosphat, Hexafluorophosphat, Hydrogencarbonat, Carbonat, Carbamat, Alkylcarbonat, Trifluormethansulfonat, Bis(trifluormethansulfonyl)imid, Nonaflat, Carboxylat, Picrat, Hennat oder Pyridoxalphosphat. Zudem sind hypergolische Anionen und oder komplexe Metallate denkbar.

In einer Ausführungsform handelt es sich bei den Anionen um Anionen welche in pharmazeutisch akzeptierbaren Salzen vorkommen. Diese sind beispielsweise in Haynes et al. Wiley InterScience 2005 und in Handbook of Pharmaceutical Salts Properties, Selection, and Use 2008 Helvetica Chimica Acta beschrieben.

In einer Ausführungsform handelt es sich bei dem separat vorliegenden Anion um eine polymerisierbare Verbindung, die eine vernetzbare und eine anionische Gruppe aufweist. Geeignete vernetzbare Gruppen umfassen ethylenische Funktionalitäten. Besonders bevorzugt sind Allylgruppen, Propargylgruppen, (Meth)acrylatgruppen oder (Meth)acrylamidgruppen und oder substituierte bzw. unsubstituierte Vinylgruppen. Geeignete anionische Gruppen umfassen die oben diskutierten Gruppen, soweit sie kovalent an einen Kohlenwasserstoff gebunden werden können. Bevorzugt sind dabei Anionen, die durch Abspaltung eines sauren Protons gebildet werden. Beispiele geeigneter Verbindungen umfassen Derivate des (Meth)acrylats oder (Meth)acrylamids, beispielsweise Derivate des (Meth)acrylats oder (Meth)acrylamids, die wenigstens eine Sulfat-, Sulfonat-, Phosphonat-, Phosphat-, Carbonat-, Carbamat-, Triflat- oder Carboxylatgruppe, insbesondere eine Sulfonatgruppe umfassen. Nicht limitierende konkrete Beispiele umfassen 2-Acrylamido-2-methylpropansulfonat, 3-Sulfopropyl(meth)acrylat, 3-sulfoethyl(meth)acrylat oder 3-(Acryloyloxy)-1-propansulfonat. In einer Ausführungsform können die polymerisierbaren Anionen als Oligomer, Präpolymer, Polymer und oder telechelisches Makromonomer vorliegen.

In einer Ausführungsform weist das Anion seinerseits eine fluorierte Gruppe auf, die beispielsweise ausgebildet sein kann, wie dies im Zusammenhang mit der fluorierten Gruppe des Tensids beschrieben wurde. Derartige Anionen haben ihrerseits oberflächenaktive Wirkung. Geeignete Beispiele umfassen Ersatzstoffe der Perfluorooctansäure (PFOA) oder Perfluorooctansulfonat (PFOS), wie beispielsweise 3H-Perfluoro-3-((3-methoxy-propoxy)propansäure).

In einer Ausführungsform handelt es sich bei dem Anion um Halogenide. lodide und Chloride sind besonders bevorzugt.

In einer Ausführungsform umfasst die Zusammensetzung ferner ein Halogenalkan, wobei das Halogenalkan vorzugsweise eine fluorierte Gruppe trägt. Auch bei der fluorierten Gruppe des Halogenalkans kann es sich beispielsweise um eine teilweise oder vollständig fluorierte Kohlenwasserstoffgruppe handeln, vorzugsweise um eine vollständig fluorierte lineare Gruppe vom Typ -(CF₂)ₙ-CF₃, wobei n zwischen 2 und 10 und vorzugsweise zwischen 4 und 8 liegt. Das Halogenalkan kann beispielsweise vom Typ X-(CH₂)ₘ-(CF₂)ₙ-CF₃ sein, wobei X ein Halogen und vorzugsweise Brom oder lod ist und m zwischen 0 und 5 liegen kann und vorzugsweise 0 ist, da die Elektronegativität der Fluor-Atome die Bildung eines Halogen-Komplexes begünstigt. Bevorzugte Halogenalkane umfassen 1-Bromoperfluorooctan, 1-lodoperfluorooctan oder 1-lodoperfluorohexan. Das Halogenalkan kann mit einem Halogenid- und vorzugsweise lodid-Anion einen sogenannten Halogen-Komplex bilden, worunter eine nicht-kovalente Wechselwirkung zwischen einem Halogen und einem Wechselwirkungspartner verstanden wird. Eine umfassende Beschreibung dieser Komplexe findet sich beispielsweise in Gilday et al., Chem. Rev. 2015, 115, 7118-7195. Darin werden neben lodalkanen auch aromatische halogenhaltige Systeme und stickstoffhaltige Verbindungen beschrieben, welche einen solchen Komplex bilden können. Dies hat gleich mehrere positive Effekte auf die Anwendung. Zum einen wird durch die Delokalisierung der negativen Ladung über die beispielsweise beiden HalogenAtome des Komplexes Elektronendichte aus der kationischen Gruppe der Fluortenside abgezogen, was zu einer stärkeren positiven Ladung führt und daher zu einer verbesserten Löslichkeit der funktionalisierten Monomere in einem organischen Lösungsmittel mit höherer Polarität bzw. höherem Dipolmoment führen kann. Des Weiteren kann durch eine Funktionalisierung des Halogenalkans mit einer fluorierten Gruppe der hydrophobe und oleophobe Effekt des Fluortensides gesteigert werden, ohne die Kettenlänge der fluorierten Gruppe in den funktionalisierten Tensiden erhöhen zu müssen. Letztlich kann das Halogenalkan auch als Reagens für weitere Reaktionen dienen, um beispielsweise mit Resten eines Photoinitiators zu reagieren.

In einer Ausführungsform handelt es sich bei dem Anion um eine zwitterionische Verbindung, vorzugsweise um ein Betain.

In einer Ausführungsform wird das Anion durch eine kovalent an das fluorhaltige Tensid gebundene anionische Gruppe gebildet. Insoweit kann es sich bei den fluorierten Tensiden um Betaine handeln. Bevorzugte anionische Gruppen umfassen dabei Sulfat, Sulfonat, Phosphonat, Phosphat, Carbonat, Trifluoromethanesulfonat, Carbamat, Triflat oder Carboxylat, insbesondere Sulfonat. Sulfonat-Anionen können beispielsweise eine gute Löslichkeit in Wasser bewirken.

In einer Ausführungsform umfasst die Zusammensetzung eine Kombination zweier oder mehrerer unterschiedlicher Anionen.

In einer Ausführungsform ist die schwefelhaltige Gruppe zwischen der kationischen Gruppe und der kovalent gebundenen anionischen Gruppe angeordnet, wobei die kovalent gebundene anionische Gruppe direkt oder mit einem zwischengelagerten Spacer an die schwefelhaltige Gruppe gebunden ist. Dabei kann die schwefelhaltige Gruppe beispielsweise direkt an das C2 der kationischen Gruppe gebunden sein.

In einer Ausführungsform ist das kovalent gebundene Anion mit einem zwischengelagerten Spacer an ein Stickstoffatom, beispielsweise an das N1 oder N3 der kationischen Gruppe gebunden.

In einer Ausführungsform ist die fluorierte Gruppe direkt oder mit einem zwischengelagerten Spacer an ein Stickstoffatom, beispielsweise an das N1 oder N3 der kationischen Gruppe gebunden.

In einer Ausführungsform ist die schwefelhaltige Gruppe zwischen der kationischen Gruppe und der fluorierten Gruppe angeordnet, wobei die fluorierte Gruppe direkt oder mit einem zwischengelagerten Spacer an die schwefelhaltige Gruppe gebunden ist. Auch dabei kann die schwefelhaltige Gruppe beispielsweise direkt an das C2 der kationischen Gruppe gebunden sein.

In einer Ausführungsform weist das Tensid eine vernetzbare Gruppe auf, sodass ein vernetzbares bzw. polymerisierbares Tensid entsteht. Geeignete vernetzbare Gruppen umfassen beispielsweise diejenigen Gruppen, die bereits im Zusammenhang mit den vernetzbaren Anionen beschrieben wurden. Es kann sich allerdings auch um Alkohole und Amine handeln, welche zum Beispiel mit Isocyanten vernetzt werden können.

In einer Ausführungsform ist die vernetzbare Gruppe direkt oder mit einem zwischengelagerten Spacer an ein Stickstoffatom, beispielsweise das N1 oder N3 der kationischen Gruppe substituiert.

In einer Ausführungsform ist die schwefelhaltige Gruppe zwischen der kationischen Gruppe und der vernetzbaren Gruppe angeordnet, wobei die vernetzbare Gruppe direkt oder mit einem zwischengelagerten Spacer an die schwefelhaltige Gruppe gebunden ist. Auch dabei kann die schwefelhaltige Gruppe beispielsweise direkt an das C2 der kationischen Gruppe gebunden sein.

In einer Ausführungsform handelt es sich bei dem Spacer um eine lineare oder verzweigte C1-C10, vorzugsweise C1-C5 und weiter vorzugsweise C2-C3 Alkylengruppe, insbesondere um Ethylen. Der Spacer ist vorzugsweise ungeladen und/oder unfluoriert. Auch Methylen oder ein Alkylen mit maximal C6 kann zum Einsatz kommen.

In einer Ausführungsform hat das Flourtensid die nachfolgend gezeigte Struktur, wobei n wie oben definiert ist:

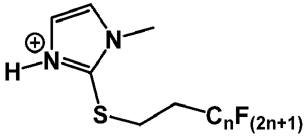

In einer Ausführungsform weisen die fluorierten Tenside zwischen 8 und 50 und vorzugsweise von zwischen 10 und 30 schwere Atome auf. Unter einem schweren Atom werden vorliegend alle Atome außer Wasserstoff verstanden.

In einer Ausführungsform weist das fluorhaltige Tensid eine der folgenden Strukturen auf: 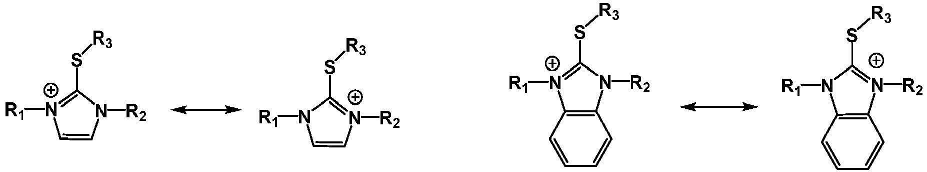 wobei einer der Reste R₁, R₂ oder R₃ eine (CH₂)₁₋₅(CF₂)₃₋₁₀CF₃ Gruppe darstellt, und die beiden weiteren der Reste unabhängig voneinander Wasserstoff oder eine substituierte oder unsubstituierte C1-C10 Alkylgruppe darstellen, die gegebenenfalls eine vernetzbare Gruppe (dabei wird die Alkylgruppe fallweise zur Alkylengruppe) oder eine kovalent gebundene anionische Gruppe aufweisen kann. Die Alkylgruppe kann dabei linear oder verzweigt sein. Geeignete substituierte Alkylgruppen umfassen Alkoxyalkyle, Aralkyle, Haloalkyle oder Alkyle mit Substituenten wie beispielsweise Sulfonamid oder N-Oxid. Beispiele geeigneter substituierter Alkylgruppen umfassen ein -(CH₂)₁₋₁₀-NH₂, ein -(CH₂)₁₋₁₀-OH und ein (CH₂)₁₋₁₀SH.

Sofern das Tensid eine vernetzbare Gruppe aufweist, kann das Tensid auch als Oligomer kovalent gebundener Einheiten vorliegen. Derartige Oligomere können beispielsweise durch Reaktionen von Alkoholen bzw. Aminen mit Isocyanaten zu den entsprechenden Urethanen bzw. Harnstoffderivaten oder durch Vernetzung von Isocyanaten oder Epoxiden erfolgen. Zudem können zum Beispiel Thiole mittels Thiol-ene-Click Reaktion zu Oligomeren bzw. Polymeren überführt werden. Ebenfalls sind Reaktionen mit an beiden Enden substituierten Alkylresten denkbar. Beispiele geeigneter Di- bzw. Multimere umfassen nicht limitierend die folgenden:

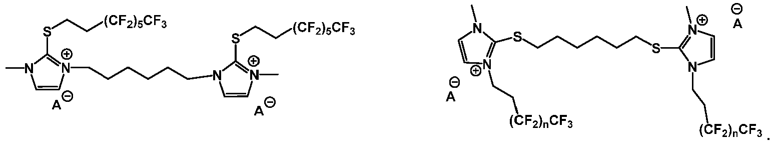

In einer Ausführungsform liegt die molare Masse der fluorierten Tenside zwischen 100 und 3500 g/mol, vorzugsweise zwischen 130 und 1000 g/mol.

In einer Ausführung kann das Tensid in der Zusammensetzung anhand der vernetzbaren Gruppe als Dimer, Trimer oder Oligomer vorliegen.

In einer Ausführung kann die Zusammensetzung neben den genannten fluorierten Tensiden weitere Tenside aufweisen.

In einer Ausführung kann es sich bei den verwendeten Fluortensiden um eine ionische Flüssigkeit handeln, die bevorzugt bei 25°C als Flüssigkeit vorliegt. Im einfachsten Fall besteht die Zusammensetzung nur aus den erfindungsgemäßen funktionalisierten Tensiden.

In anderen Ausführungsformen wird das Tensid mit einem geeigneten Lösungsmittel für die jeweilige Anwendung verdünnt, und anschließend in die für die jeweilige Anwendung vorgesehene Zusammensetzung eingebracht. Das bevorzugte Lösungsmittel ist Wasser, allerdings sind andere polare und apolare Lösungsmittel oder auch ionische Flüssigkeiten als potentielle Lösungsmittel geeignet.

In einer Ausführungsform umfasst die Zusammensetzung zwischen 0,001 und 99,999 Gew.-% Wasser.

In einer Ausführungsform umfasst die Zusammensetzung zwischen 0,001 und 100 Gew.-% des fluorierten Tensids.

Die Tenside der erfindungsgemäßen Zusammensetzungen haben hervorragende Löslichkeitsprofile und antistatische Eigenschaften. Diese können je nach Anwendung sowohl durch die Wahl der Substituenten, die Gestalt der Fluorkette(n) (vollständig fluorierte Kette, Fluoroether als Kette, Verzweigte Kette(n), Kettenlänge) und das Anion gesteuert werden. Deshalb besteht eine hohe Flexibilität sowohl in der Synthese als auch eine breite Einsatzmöglichkeit in der Anwendung.

Vor dem eingangs genannten Hintergrund betrifft die Erfindung ferner die Verwendung der erfindungsgemäßen Zusammensetzung als oberflächenaktives Mittel. Denkbar ist der Einsatz zur Verringerung der Oberflächenspannung, als Entschäumer, zur Einstellung optischer Eigenschaften oder zur Einstellung der Sauerstofflöslichkeit oder Sauerstoffpermeabilität. In einer Ausführungsform wird die Zusammensetzung in Oberflächenbeschichtungen, Lacken, Löschmitteln, Hydrogelen und/oder bei der Polymerisation von Fluoropolymeren verwendet. Auf die im einleitenden Teil der Patentbeschreibung genannten Anwendungen wird ferner verwiesen.

Letztlich betrifft die Erfindung ein Verfahren zur Herstellung der erfindungsgemäßen Zusammensetzung. Ein derartiges Verfahren ist beispielsweise der Schritt einer Umsetzung einer ungeladenen Verbindung mit einer N-substituierten und vorzugsweise N-alkylierten heterozyklischen Gruppe, einer zwei- oder mehrwertigen schwefelhaltigen Gruppe und einer fluorierten Gruppe mit einer Säure. Alternativ oder zusätzlich ist auch eine Quaternisierung, Metathese oder dergleichen vorgesehen wie sich dies aus der Beschreibung der Ausführungsbeispiele ergibt.

Weitere Einzelheiten und Vorteile der Erfindung ergeben sich aus den nachfolgend unter anderem anhand der Figuren beschriebenen Ausführungsbeispielen. In den Figuren zeigen:
- Figur 1:: eine Reaktionsgleichung für die Synthese eines polymerisierbaren fluorhaltigen Tensids;
- Figur 2:: eine Reaktionsgleichung für die Synthese eines fluorhaltigen Tensids aus Thiamazol;
- Figur 3:: eine Reaktionsgleichung für die Synthese eines fluorhaltigen Tensids aus einer Fluorbase mit einer Säure;
- Figur 4:: eine schematische Reaktionsgleichung für die Synthese eines fluorhaltigen Tensids durch Metathese;
- Figur 5:: eine Reaktionsgleichung für die Synthese eines fluorhaltigen Tensids durch Thionisierung und Alkylierung;
- Figur 6:: eine fotographische Abbildung, welche die schaumhemmende Wirkung von 1-Methyl-2-((perfluorohexylethyl)thio)imidazolium-O,S-dimethyl phosphorothioat zeigt;
- Figur 7:: eine fotographische Abbildung, welche die schaumerzeugende Wirkung von 3-(1-Methyl-2-((1H,1H,2H,2H-perfluorooctyl)thio)imidazolium-3-yl) propan-1-sulfonat zeigt;
- Figur 8:: eine Darstellung der Kristallstruktur von 3-(1-Methyl-2-((perfluorohexylethyl)thio)imidazolium-3-yl)propan-1-sulfonat; und
- Figur 9:: eine Darstellung der Kristallstruktur von 1-Methyl-2-((perfluorohexylethyl)thio)imidazoliumiodid-Co-1-lodoperfluorooctylan.

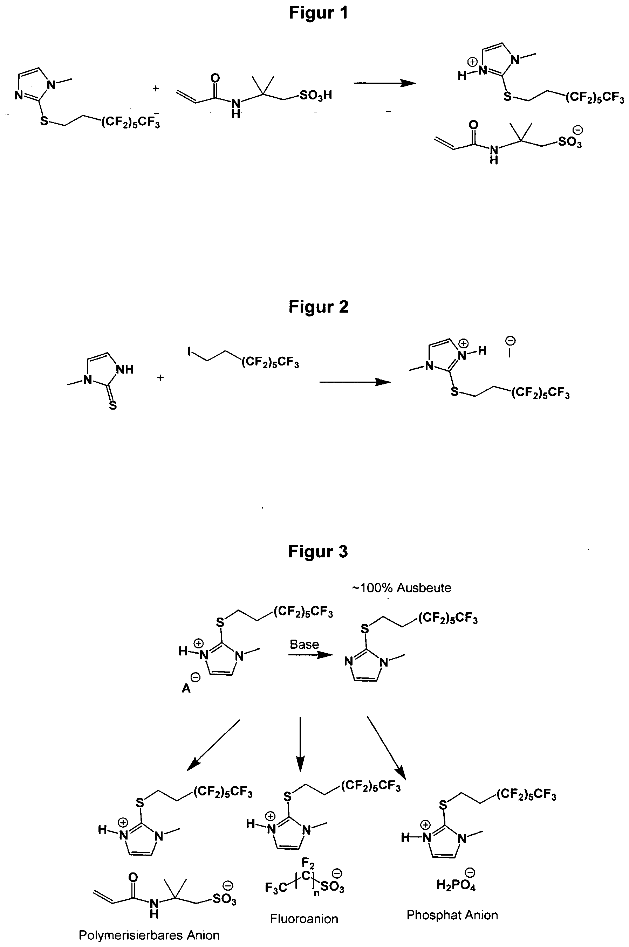

Eine im Rahmen der vorliegenden Erfindung bevorzugte fluorhaltige Verbindung, welche als Tensid wirkt und zusätzlich eine vernetzbare Funktionalität enthält, ist in Figur 1 mit 2-Acrylamido-2-methylpropansulfonat als Gegenion dargestellt. Das Anion kann durch die Acrylamid-Funktionalität polymerisiert werden.

Figur 2 zeigt eine Reaktionsgleichung für die Synthese eines erfindungsgemäßen fluorhaltigen Tensids aus Thiamazol. Diese Alkylierung gelingt in besonders guten Ausbeuten und ist mit geringen Kosten verbunden.

Die Reaktionsgleichung gemäß Figur 3 steht repräsentativ für einen allgemeinen synthetischen Zugang zu erfindungsgemäßen fluorhaltigen Tensiden. Dabei kann ausgehend von einem erfindungsgemäßen fluorhaltigen Tensid durch einfache Deprotonierung mittels Base, bevorzugt mittels Carbonat, eine ungeladene fluorhaltige Base erzeugt werden, welche anschließend mit einer Säure in hohen Ausbeuten wiederum zu alternativen fluorhaltigen Tensiden führt. Die Ausbeuten über alle Syntheseschritte liegen dabei für gewöhnlich über 90%.

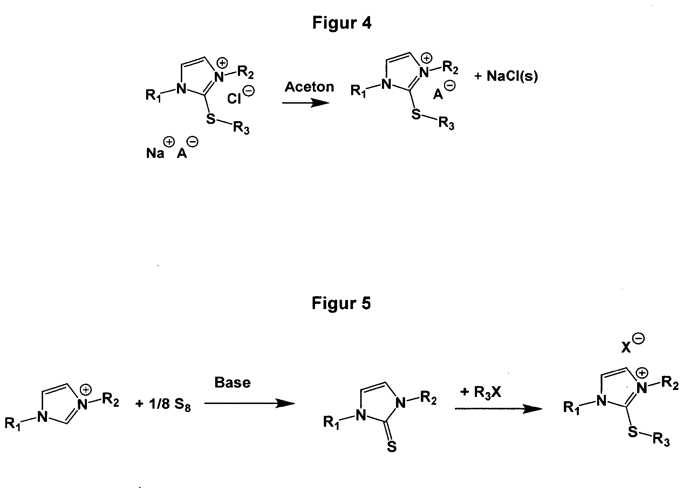

Figur 4 zeigt eine Metathese-Reaktion eines erfindungsgemäßen fluorhaltigen Tensids. Die Bezeichnung Kat⁺ steht dabei für ein Kation. Eine bevorzugte Methathese stellt zum Beispiel das Ausfällen von Natriumchlorid in Aceton bei gleichzeitiger Bildung eines neuen Fluortensides dar.

Figur 5 beschreibt einen allgemeinen synthetischen Zugang zu erfindungsgemäßen Tensiden aus disubstituiertem Imidazol als Vorstufe.

Allgemein ist zu erwähnen, dass protonierte erfindungsgemäße Tenside je nach pH wieder deprotoniert werden können. Dies kann bei einigen Anwendungen gezielt eingesetzt werden. Demgegenüber sind disubstituierte erfindungsgemäße Fluortenside auch bei basischem pH relativ stabil. Bei extremen pH-Bedingungen kann bei den dialkylierten Systemen eine Beta-H Elimination und daraufhin eine Eliminierung von z.B. 1,H,1H,2H,2H-Perfluorooct-1-en erfolgen. Dies kann in bestimmten Fällen einen Abbau begünstigen bzw. erleichtern. Dabei kann die kationische Ladung gezielt verwendet werden, um Abwässer zum Beispiel über lonenaustauscher vorzugsweise im sauren oder neutralen Milieu zu filtern. Anschließend kann oben genannte Abbaureaktion verwendet werden um eine Entfernung der Fluorseitenkette zu erreichen. Dies ermöglicht einen besonders umweltverträglichen Prozess.

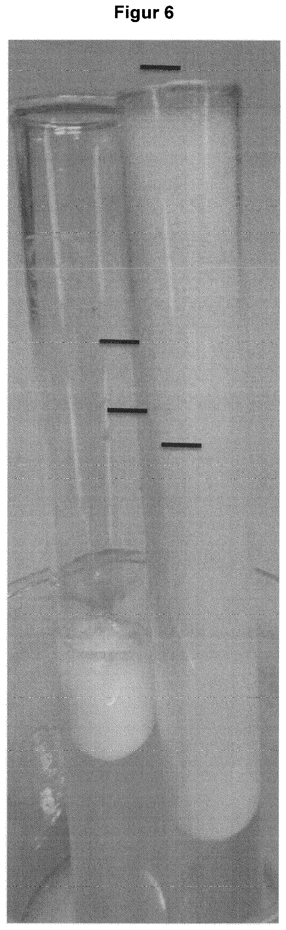

Das Bild gemäß Figur 6 beruht auf dem Einsatz des im unten beschriebenen Synthesebeispiel 5 beschriebenen 1-Methyl-2-((perfluorohexylethyl)thio) imidazoliumO,S-dimethylphosphorothioats als Entschäumer. Für die Aufnahme dieses Fotos wurden Wasser und eine handelsübliche Seife in beiden Reagenzgläsern in gleichen Mengen gemischt. Anschließend wurde in das linke Reagenzglas das erfindungsgemäße Fluortensid gegeben. Danach wurden beide Reagenzgläser für 1 Minute geschüttelt. Nachdem die Reagenzgläser 10 Sekunden ruhten, wurde das Foto aufgenommen. Die Hemmung der Schaumbildung durch das erfindungsgemäße Tensid ist gut erkennbar.

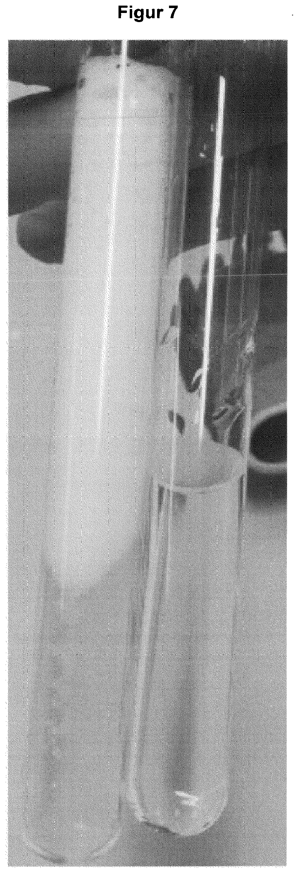

Figur 7 zeigt ein Foto, in dem links ein Reagenzglas zu sehen ist, das mit 10 ml Wasser gefüllt ist, in dem 20 mg 3-(1-Methyl-2-((1H,1H,2H,2H-perfluorooctyl)thio)imidazolium-3-yl) propan-1-sulfonat gelöst wurden. Rechts ist ein Reagenzglas mit destilliertem Wasser zu sehen. Beide Reagenzgläser wurden für 1 Minute geschüttelt. Dabei ist eine deutliche Schaumbildung durch das Tensid zu beobachten.

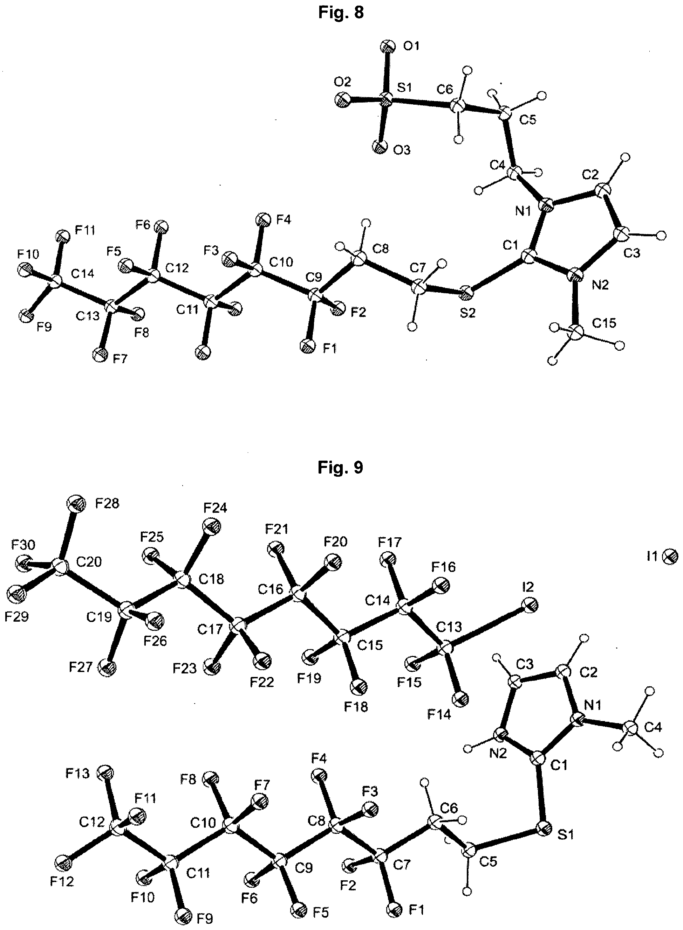

Figur 8 zeigt die aus Einkristallröntgenstrukturanalyse ermittelte Molekülstruktur des für die Aufnahme des Fotos gemäß Figur 7 verwendeten erfindungsgemäßen Fluortensides.

Figur 9 zeigt einen Halogenkomplex zwischen einem erfindungsgemäßen Fluortensid und 1-lodoperfluorooctan.

### Synthesebeispiel 1:

### Synthese von 1-Methyl-2-((1H,1H,2H,2H-perfluorooctyl)thio)-1H-imidazolium-2-(methacryloyloxy)ethan-1-sulfonat

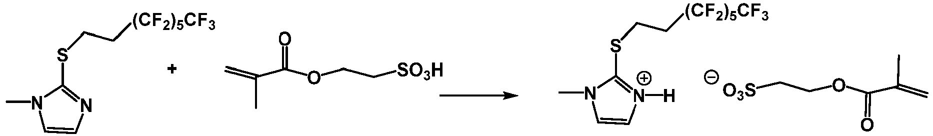

### Ansatz:

3,067 g (6,66 mmol) 1-Methyl-2-((1H, 1H, 2H, 2H-Perfluorooctyl)thio)-1H-imidazol 1,267 g (6,52 mmol) 2-Methacryloylethan-1-sulfonsäure

### Durchführung:

3,067 g (6,66 mmol) 1-Methyl-2-((1H, 1H, 2H, 2H-perfluorooctyl)thio)-1H-imidazol und 1,267 g (6,52 mmol) 2-Methacrylamidoethan-1-sulfonsäure wurden ohne Lösungsmittel zusammengegeben, dabei kam es zur sofortigen Reaktion und Präzipitation des Produkts. Für einen vollständigen Umsatz wurde die Mischung im Ultraschallbad in 20 ml Methanol gelöst. Das Lösungsmittel wurde am Rotavapor entfernt. Das flüssige Produkt wurde mit Ether gewaschen, dabei fiel ein weißes Pulver aus, welches abfiltriert und dreimal mit Ether gewaschen wurde.

### Ausbeute:

3,867 g 1-Methyl-2-((1H,1H,2H,2H-perfluorooctyl)thio)-1H-imidazolium-2-(methacryloyloxy)ethan-1-sulfonat (5,91 mmol, 90,5%)
¹H-NMR (300 MHz; 25°C; CD₃OD):
7,73 (I=1; d (J3=3Hz); CH-Imidazolium); 7,69 (I=1; d (J3=3Hz); CH-Imidazolium); 6,12 (I=1; m; CH₂= Trans); 5,61 (I=1; m; CH₂= cis); 4,49 (I=2; t; CH₂-S); 3,96 (3H; s; CH₃-N); 3,43 (I=2; t; CH₂-NH); 3,17 (I=2; t; CH₂-SO₃-); 2,68 (I=2; m; CH₂-CF₂ ); 1,92(I=3; m; CH₃-C);
¹³C-NMR (75 MHz; 25°C; CD₃OD):
168,71 (C=O); 140,94(C=); 137,71 (C-S); 127,35 (CH-N); 126,57 (CH₂=); 122,70 (CH-N); 61,73 (CH₂-SO₃⁻); 51,33 (CH₂-N); 36,18(CH₃-N); 32,65(t; CH₂-CF₂); 27,76(t; CH₂-S); 18,51 (CH₃);
Schmelzpunkt: 88,6 - 89,9°C

### Bestimmung der Löslichkeit:

In 5 ml Wasser wurden 3,84 g 1-Methyl-2-((1H, 1H,2H,2H-perfluorooctyl)thio)-1H-imidazolium-2-(methacryloyloxy)ethan-1-sulfonat gegeben. Die Suspension wurde für 5 Minuten ins Ultraschallbad gegeben und für 20 Minuten zentrifugiert, Danach wurde der wässrige Überstand gewogen und für 5 Minuten mit Argon gespült um möglicherweise gelösten Sauerstoff zu entfernen. Die wässrige, leicht gelbe Lösung wog 7,126 g. Der Versuch wurde bei ca. 23°C durchgeführt.

Aus diesen Daten ergibt sich eine Löslichkeit von ca. 430 g/L Wasser.

### Synthesebeispiel 2:

### Synthese von 1-Methyl-2-((1H,1H,2H,2H-perfluorooctyl)thio)-1H-imidazolium-2-acrylamido-2-methylpropansulfonat

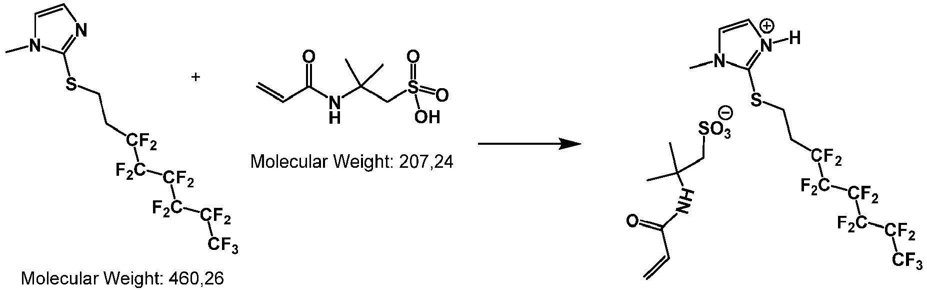

### Theoretische Ausbeute:

2,67 g von 1-Methyl-2-((1H, 1H,2H,2H-perfluorooctyl)thio)-1H-imidazolium-2-acrylamido-2-methylpropan sulfonat.

### Durchführung:

0,829 g (4 mmol) 2-Acrylamido-2-methylpropansulfonsäure wurden in 30 ml Methanol gelöst und nach 10 Minuten rühren wurden 1,841 g (4 mmol) 1-Methyl-2-((1H, 1H, 2H, 2H-Perfluorooctyl)thio)-1H-imidazol in 10 ml Methanol langsam zu getropft. Die Mischung wurde für einen Tag bei Raumtemperatur gerührt und das Methanol anschließend teilweise am Rotationsverdampfer abgezogen. Danach wurde 2 mal mit Hexan gewaschen, die Hexanphase abdekantiert und anschließend das restliche Lösungsmittel am Rotationsverdampfer entfernt. Es wurde ein weißes, leicht gelbliches Produkt erhalten.

### Ausbeute:

2,24g (83,9% d.Th)

### Auswertung und Charakterisierung:

¹H NMR (300 MHz, CD₃OD) δ 7.75 (I=1; d, CH-Imidazolium), 7.70 (I=1; d, CH-Imidazolium), 6.25 bis 6.07 (I=2; m; Trans-CH₂= und CH-C=O) , 5.58 und 5.55 (I=1; dxd; Cis-CH₂=), 3.96 (I=3; s; CH₃-N), 3.44 (I=2, t, CH2-S), 3.24(I=2,s, C-CH₂-SO₃), 2.78 bis 2.60 (I=2, m, CH₂-CF₂), 1.57 (I=6, s, 2xCH₃).

¹³C NMR (75 MHz, CD₃OD) δ 167.56 (C=O), 140.93 (C2), 133.69 (CH-Vinyl), 127.35 (CH₂-Vinyl), 125.73 (CH-Imidazolium), 122.68 (CH-Imidazolium), 60.28 (CH₂-SO₃), 53.60 (CH₂-Amid), 36.21 (CH₃-N), 32.63("txt" , CH₂-CF₂), 27.78 ("t", 27.72 CH₂-S), 27.19 (2x CH₃).

Dieses Tensid ist polymerisierbar und wasserlöslich.

### Verwendung als Emulgator von Teflon:

Es wurde eine Lösung aus 10 ml Wasser, 0,100 g 1-Methyl-2-((1H,1H,2H,2H-perfluorooctyl)thio)-1H-imidazolium-2-acrylamido-2-methylpropansulfonat wurde in 10 ml Wasser gelöst und anschließend wurden zu dieser Lösung 0,200 g Teflon 1 µm (Sigma Aldrich) zugegeben. Dabei bildete sich eine Suspension, die für mindestens 4 Stunden stabil blieb. Nach 2 Tagen hatte sich ein weißes Pulver am Boden abgesetzt.

### Synthesebeispiel 3:

### Synthese von 1-Methyl-2-((1H,1H,2H,2H-perfluorooctyl)thio)imidazoliumiodid

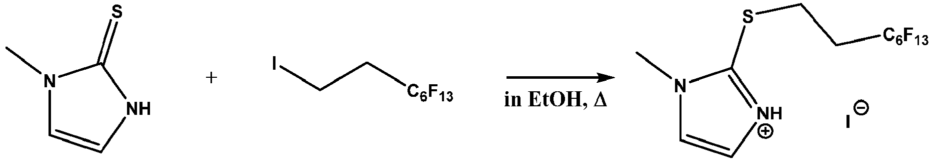

100 g (0,876 mol) 1-Methyl-1,3-dihydro-2*H*-imidazol-2-thion (Thiamazol) und 500 g (1,05 mol) 1H,1H,2H,2H-Perfluorooctyliodid wurden unter leichtem Erhitzen in 500 ml Ethanol gelöst, die resultierende Reaktionslösung anschließend für 36 Stunden refluxiert. Danach wurde die Lösung mittels eines Rotationsverdampfers so lange eingeengt, bis exzessives Schäumen ein weiteres Einengen stark erschwerte. Diese gesättigte Lösung wurde dann über Nacht bei Raumtemperatur gelagert, um das Produkt kristallisieren zu lassen. Um die Präzipitation des Produktes zu vervollständigen und überschüssiges 1H, 1H,2H,2H-Perfluorooctyliodid zu entfernen, wurden zum präzipitierten Produkt 500 ml Diethylether zugegeben. Die Mischung wurde für einige Minuten geschüttelt, dann das Produkt abfiltriert und mit weiteren 300 ml Diethylether gewaschen; die Trocknung des Produktes erfolgte für 24 Stunden im Hochvakuum. Es wurden 500 g an 1-Methyl-2-((1H,1H,2H,2H-perfluorooctyl)thio)imidazoliumiodid isoliert (97% d. Th.).
¹³C NMR (75 MHz, CDCl₃) δ 140.65 (s, C(1)), 124.58 (s, C(3)), 121.72 (s, C(2)), 126-100 (m, Perfluorohexyl), 36.21 (s, C(4)), 31.39 (t, C(6)), 27.88 (t, C(5)).

¹H NMR (300 MHz, CDCl₃) δ 10.81 (1H, s(br), NH Imidazolium, Anm.: die chemische Verschiebung dieses Signals scheint stark konzentrationsabhängig zu sein), 7.50 (1H, d, C(3)H Imidazolium), 7.44 (1H, d, C(2)H Imidazolium), 3.91 (3H, s, CH₃), 3.76 (2H, t, CH₂-S), 2.61 (2H, tt, CH₂-CF₂).
Löslichkeit in MeOH: 1300 g/l
Löslichkeit in DMSO: 740 g/l
Löslichkeit in 3M™ Novec™ 71 IPA: 9,6 g/l
Löslichkeit in 3M™ Novec™ 71 IPA als 1-lodoperfluorooctyl-Co-Komplex: 20,5 g/l

### Synthesebeispiel 4:

### Synthese von 1-Methyl-2-((1H,1H,2H,2H-perfluorooctyl)thio)imidazolium-hexafluoro acetylacetonat

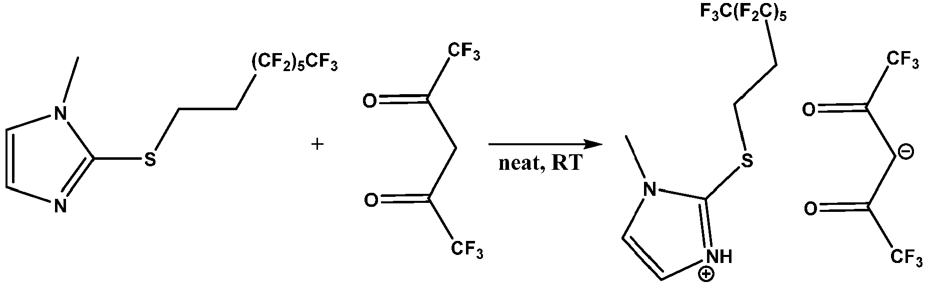

1,04 g (5,0 mmol) Hexafluoroacetylaceton wurden zu 2,30 g (5,0 mmol) 1-Methyl-2-((perfluorohexylethyl)thio)imidazol zugetropft, die resultierende Reaktionsmischung dann für 2 Minuten leicht geschüttelt. Das Produkt stellte eine etwas viskose, farblose Flüssigkeit dar; die Ausbeute ist quantitativ.

¹³C NMR (75 MHz, Aceton-d6) δ 174.32 (q, Carbonyl-C Hexafluoroacetylacetonat), 139.24 (s, C(1)), 124.34 (s, C(3)), 122.92 (s, C(2)), 118.18 (q, CF₃ Hexafluoroacetylacetonat), 126-100 (m, Perfluorohexyl), 85.64 (s(br), Carbanion Hexafluoroacetylacetonat), 34.27 (s, C(4)), 31.22 (t, C(6)), 26.32 (t, C(5)).

¹H NMR (300 MHz, Aceton-d6) δ 15.83 (1H, s(breit), NH Imidazolium), 7.63 (1H, d, C(3)H Imidazolium), 7.40 (1H, d, C(2)H Imidazolium), 5.85 (1H, s(breit), CH Hexafluoroacetylacetonat), 3.87 (3H, s, CH₃), 3.55 (2H, t, CH₂-S), 2.59 (2H, tt, CH₂-CF₂).

### Synthesebeispiel 5:

### Synthese von 1-Methyl-2-((1H,1H,2H,2H-perfluorooctyl)thio)imidazolium-O,S-dimethylphosphorothioat

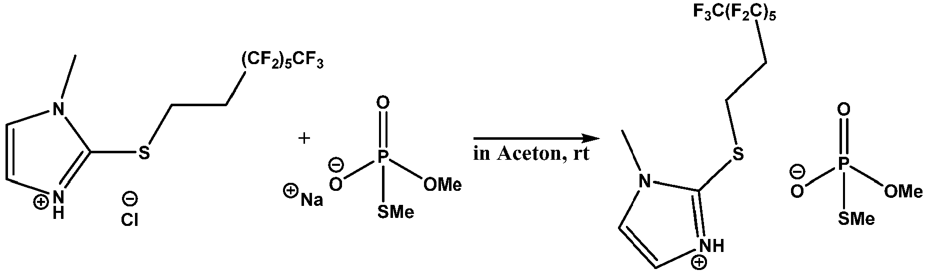

Zu einer Dispersion von 2,48 g (5,0 mmol) 1-Methyl-2-((1H,1H,2H,2H-perfluorooctyl)thio)imidazoliumchlorid in 25 ml Aceton wurde eine Lösung von 0,821 g (5,0 mmol) Natrium-O,S-dimethylphosphorothioat in 25 ml Aceton unter Rühren zugetropft. Die resultierende Reaktionsmischung wurde über Nacht bei Raumtemperatur gerührt, anschließend das entstandene Natriumchlorid abfiltriert. Das Lösungsmittel des Filtrats wurde im Rotationsverdampfer entfernt, übrig blieb ein farbloses, viskoses Öl. Dieses wurde über Nacht im Hochvakuum getrocknet, es ergaben sich 2,86 g an 1-Methyl-2-((1H,1H,2H,2H-perfluorooctyl)thio)imidazolium-O,S-dimethyl phosphorothioat (95% d. Th.).

¹³C NMR (75 MHz, Aceton-d6) δ 139.73 (s, C(1)), 126.11 (s, C(3)), 124.20 (s, C(2)), 126-100 (m, Perfluorohexyl), 52.71 (d, O-CH₃ O,S-dimethylphosphorothioat), 35.27 (s, C(4)), 32.05 (t, C(6)), 26.62 (t, C(5)), 12.55 (s, S-CH₃ O,S-dimethylphosphorothioat).

¹H NMR (300 MHz, Aceton-d6) δ 12.31 (1H, s(breit), NH Imidazolium), 7.79 (1H, d, C(3)H Imidazolium), 7.49 (1H, d, C(2)H Imidazolium), 3.93 (3H, s, CH₃), 3.56 (5H, scheinbar s(br), CH₂-S und O-CH₃ O,S-dimethylphosphorothioat), 2.71 (2H, tt, CH₂-CF₂), 2.16 (3H, s, S-CH₃ O,S-dimethylphosphorothioat).

Diese Verbindung wurde zu einer Lösung aus Wasser und kommerziell erhältlicher Seife gegeben. Als Referenz wurde ein Reagenzglas mit Wasser und Seife ohne Tensid verwendet. Beide Reagenzgläser wurden für 1 Minute geschüttelt und nach 10 Sekunden fotografiert (Fig. 6). Dabei kann beobachtet werden, dass sich durch das Fluortensid deutlich weniger Schaum bildete.

### Synthesebeispiel 6:

### Synthese von 3-(1-Methyl-2-((1H,1H,2H,2H-perfluorooctyl)thio)imidazolium-3-yl) propan-1-sulfonat

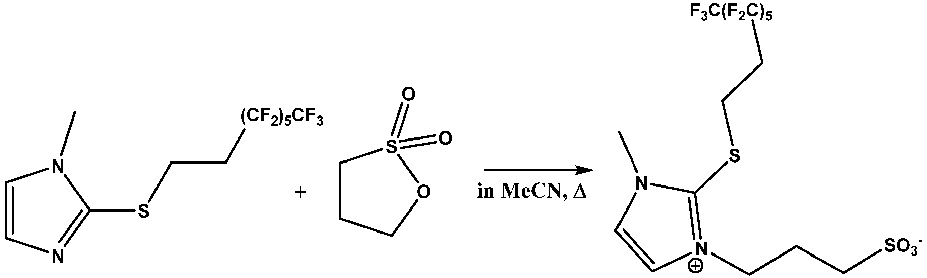

3 g (6,52 mmol) 3-(1-Methyl-2-((1H,1H,2H,2H-perfluorooctyl)thio)imidazol und 0,92 g (7,5 mmol) 1,3-Propansulton wurden in 5 ml Acetonitril gelöst und für 16 Stunden refluxiert, wobei schon während dieser Zeit einiges an Produkt präzipitierte. Nach Abkühlen des Reaktionsgemisches wurde die Präzipitation des Produktes durch Zugabe von 50 ml Diethylether und Lagerung des Gemisches bei -20 °C für einige Stunden komplettiert. Anschließend wurde das Produkt abfiltriert und 2-mal mit je 30 ml Diethylether gewaschen, dann über Nacht am Hochvakuum getrocknet. 3,56 g an weißem, pulvrigem Produkt konnten isoliert werden (94% d. Th.).
¹³C NMR (75 MHz, CD₃OD) δ 140.66 (s, C(1)), 127.14 (s, C(3)), 125.63 (s, C(2)), 126-100 (m, Perfluorohexyl), 49.70 (s, C(6)), 48.66 (s, C(4)), 37.06 (s, C(15)), 32.04 (t, C(8)), 27.96 (t, C(7)), 27.13 (s, C(5)).
¹H NMR (300 MHz, CD₃OD) δ 8.00 (1H, d, C(3)H Imidazolium), 7.89 (1H, d, C(2)H Imidazolium), 4.65 (2H, t, C(4)H₂), 4.09 (3H, s, CH₃), 3.41 (2H, t, CH₂-S), 2.93 (2H, t, C(6)H₂), 2.79 (2H, tt, CH₂-CF₂), 2.39 (2H, quin, C(5)H₂).

Die Wasserlöslichkeit beträgt ca. 160 g/l.

Es wurden ca. 20 mg dieser Verbindung zu 10 ml Wasser zugegeben. Als Referenz wurde ein Reagenzglas mit Wasser und ohne Tensid verwendet. Beide Reagenzgläser wurden für 1 Minute geschüttelt und nach 10 Sekunden fotografiert (Fig. 7). Dabei kann beobachtet werden, dass dieses Fluortensid als Schaumbildner aktiv ist.

### Synthesebeispiel 7:

### Synthese von 3-((3-Octyl-1-(1H,1H,2H,2H-perfluorooctyl)imidazolium-2-yl)thio)propan-1-sulfonat

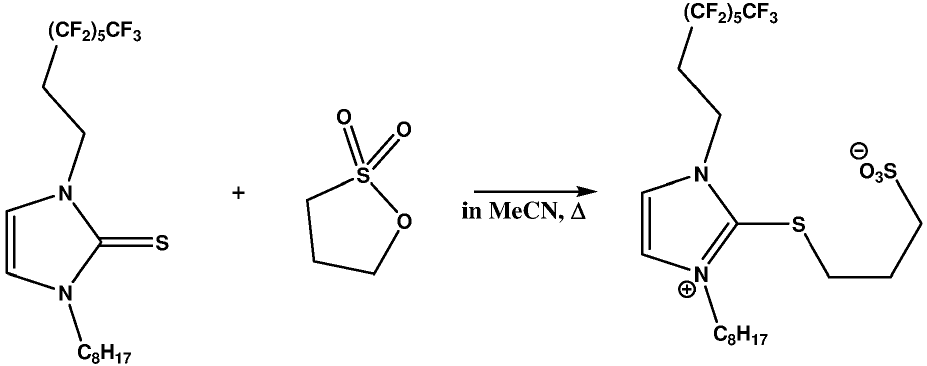

55,8 g (100 mmol) 3-octyl-1-(1H,1H,2H,2H-perfluorooctyl)-1,3-dihydro-2H-imidazol-2-thion und 15,9 g (130 mmol) 1,3-Propansulton wurden mit 65 ml Acetonitril versetzt und für 16 Stunden vorsichtig refluxiert (starke Schaumbildung). Nach Abkühlen der Reaktionsmischung wurden zur Produktpräzipitation 300 ml Diethylether zugegeben und dieses Gemisch dann noch über Nacht bei -20 °C gelagert. Anschließend wurde das Produkt abfiltriert und 2-mal mit 150 ml Diethylether gewaschen, dann für 24 Stunden am Hochvakuum getrocknet. Es ergaben sich 37,5 g an weißlichem, pulvrigem Produkt (55% d. Th.).
¹³C NMR (75 MHz, CD₃OD) δ 141.51 (s, C(1)), 125.96 (s, C(3)), 125.89 (s, C(2)), 126-100 (m, Perfluorohexyl), 51.23 (s, C(4)), 50.36 (s, C(22)), 43.15 (t, C(12)), 36.56 (s, C(20)), 32.85 (s, C(5)), 31.61 (t, C(13)), 31.07 (s, C(6)), 30.21 (s, C(7)), 30.10 (s, C(8)), 27.24 (s, C(9)), 27.00 (s, C(21)), 23.64 (s, C(10)), 14.34 (s, C(11)).
¹H NMR (300 MHz, CD₃OD) δ 8.06 (1H, d, C(3)H Imidazolium), 7.98 (1H, C(2)H Imidazolium), 4.82 (2H, t, C(124)H₂-N), 4.43 (2H, t, C(4)H₂-N), 3.29 (2H, t, C(20)H₂-S), 3.01 (2H, tt, CH₂-CF₂), 2.91 (2H, t, C(22)H₂-SO₃⁻), 2.09 (2H, quin, C(5)H₂), 1.93 (2H, quin, C(21)H₂), 1.33 (10H, m, C(6-10)H₂), 0.89 (3H, t, C(11)H₃).

Werte für die Löslichkeit des Produkts in unterschiedlichen Lösungsmitteln sind in der nachfolgenden Tabelle 1 wiedergegeben.

**Tabelle 1**

| Lösungsmittel | Löslichkeit (g/l) |
|---|---|
| Et₂O | 0,5 |
| Aceton | 3,0 |
| MeCN | 9,0 |
| EtOH | 163 |
| H₂O | 2,0 |

### Beispiel 8:

### Messung von Oberflächenspannungen:

Die Oberflächenspannung wurde am hängenden Tropfen mittels eines *"Drop Shape Analyser DSA 25"* der Firma Krüss bestimmt. Das Tropfenvolumen betrug bei den meisten Messungen ca. 5 µl. Die Messungen wurden bei Temperaturen zwischen 24,5 und 25,1 °C und einer Luftfeuchtigkeit von ca. 19% durchgeführt. Jede Probe wurde insgesamt 10-mal gemessen. Die Standardabweichungen sind in der unten angeführten Tabelle mit s gekennzeichnet. Bei den Messungen bei einer Konzentration von 0,05 Gew.-% Tensid wurden zum Teil größere Tropfenvolumen verwendet, um eine bessere Reproduzierbarkeit der Messergebnisse zu erhalten. Die Messwerte sind in der nachfolgenden Tabelle 2 wiedergegeben.

**Tabelle 2**

| | Konzentration Tensid in Wasser 0.5 Gew.-% | | Konzentration Tensid in Wasser 0.05 Gew.-% | |
|---|---|---|---|---|
| | n=10 | | n=10 | |
| | OFS (mN/m) | s | OFS (mN/m) | s |
| 1-Methyl-2-((1 H,1H,2H,2H-perfluorooctyl)thio)-1H-imidazolium-2-(methacryloyloxy)ethan-1-sulfonat | 16,22 | 0,05 | 23,24 | 0,21 |
| 3-(1-Methyl-2-((perfluorohexylethyl)thio)-imidazolium-3-yl)-propan-1-sulfonat | 24,94 | 0,09 | 29,74* | 0,16 |
| Perfluorooctansäure | 16 | 0,34 | 55,68** | 0,26 |
| Ammonium-4,8-dioxa-3H-perfluorononanoat | 46,42 | 0,29 | 64,87** | 0,07 |
| 1-Methyl-2-((1H, 1H, 2H, 2H-Perfluorooctyl)thio)-1H-imidazolium-methansulfonat | 16,21 | 0,06 | 24,39 | 0,14 |

| | | | | |
|---|---|---|---|---|
| * Probenvolumen betrug 8 µl; ** Probenvolumen betrug 20 µl. | | | | |

Von 1-Methyl-2-((1H,1H,2H,2H-perfluorooctyl)thio)-1H-imidazolium-2-(methacryloyloxy)ethan-1-sulfonat wurden noch weitere Messungen der Oberflächenspannung bei unterschiedlichen Konzentrationen nach derselben Methode wie oben beschrieben durchgeführt. Das Ergebnis ist in untenstehender Tabelle 3 zusammengefasst:

**Tabelle 3**

| c (%) | OFS (mN/m) | s |
|---|---|---|
| 0,5 | 16,22 | 0,05 |
| 0,1 | 20,8 | 0,09 |
| 0,05 | 23,24 | 0,21 |
| 0,01 | 32,11 | 0,29 |
| 0,005 | 70,28 | 0,2 |

Bei einer Konzentration von 0,5 Gew.-% in Wasser sind die Werte der erfindungsgemäßen Tenside mit dem Stand der Technik vergleichbar. Bei niedrigeren Tensidkonzentrationen wird dieser aber bei weitem übertroffen. Zudem kann 1-Methyl-2-((1H,1H,2H,2H-perfluorooctyl)thio)-1H-imidazolium-2-(methacryloyloxy)ethan-1-sulfonat noch polymerisiert werden, was zum Beispiel in Lacken zu einer zusätzlich erhöhten Umweltverträglichkeit führen kann.

### Beispiel 9:

### Synthese von Hydrogelen:

Unterschiedliche Hydrogele wurden synthetisiert. Die Ausgangsprodukte sind in der nachfolgenden Tabelle 4 gelistet.

**Tabelle 4**

| | Referenz | Hydrogel 1 | Hydrogel 2 | Hydrogel 3 | Hydrogel 4 |
|---|---|---|---|---|---|
| Natrium 2-Acrylamido-2-methanpropansulfonat | 48,00 | 39,18 | 43,20 | 43,20 | 39,18 |
| Wasser | 48,00 | 39,18 | 43,20 | 43,20 | 39,18 |
| Ethylenglycoldimethacrylat | 2,96 | 2,42 | 2,66 | 2,66 | 2,42 |
| 2-Hydroxy-2-methyl-1-phenyl-1-propanon | 1,04 | 0,85 | 0,94 | 0,94 | 0,85 |
| 3-((1-(1H,1H,2H,2H-Perfluorooctyl)-3-vinyl-1 H-imidazolium-2-yl)thio)propan-1-sulfonat | --- | 18,37 | 10,00 | --- | --- |
| 1-Methyl-2-((1 H,1H,2H,2H-perfluorooctyl)thio)-1 H-imidazolium 2-acrylamido-2-methylpropan sulfonat | --- | --- | --- | 10,00 | 20,00 |
| Einwaage [g] | 2,72 | 2,64 | 2,29 | 2,26 | 3,65 |
| In 800 ml Wasser für 13 Stunden gequollen | 77,69 | 21,72 | 27,59 | 32,26 | 47,55 |
| Verhältnis | 28,56 | 8,23 | 12,05 | 14,27 | 13,03 |
| | transparent | transparent | transparent | trüb | weiß |

Die Beispiele zeigen, dass sich die verwendeten Fluortenside zum Einbau in ein Hydrogel einigen. Weiters wurde dabei das Quellverhalten verändert, blieb aber trotz eines hohen Fluorgehaltes überraschend hoch. Bemerkenswert sind vor allem die Hydrogele 1 und 2, welche trotz der hohen Wasseraufnahme immer noch transparent blieben. Dies könnte besonders in Kontaktlinsen von Vorteil sein. Mit einem hohen Fluorgehalt in Hydrogelen soll einerseits der Brechungsindex des Hydrogels und anderseits die Sauerstofflöslichkeit bzw. Sauerstoffdurchlässigkeit gezielt verändert werden können.

Die erfindungsgemäßen Fluortenside sind unter anderem auch für den Einsatz im wässrigen Medium gedacht. Dabei kann eine gute Wasserlöslichkeit von großem Interesse sein. Manche der erfindungsgemäßen Zusammensetzungen zeigten dabei eine überraschende hohe Wasserlöslichkeit. Die unten angegebene Tabelle 5 fasst einige gemessene Werte zusammen. Die Werte sind nicht exakt und dienen nur zum besseren Verständnis.

**Tabelle 5**

| Substanz | Löslichkeit in Wasser [g/l] |
|---|---|
| 1-Methyl-2-((1 H,1H,2H,2H-perfluorooctyl)thio )im idazoliumchlorid | 1750 |
| 1-Methyl-2-((1 H,1H,2H,2H-perfluorooctyl)thio)im idazoliumbromid | 1200 |
| 1-Methyl-2-((1 H,1H,2H,2H-perfluorooctyl)thio)im idazoliumiodid | < 1 |
| 3-(1-Methyl-2-((1 H,1H,2H,2H-perfluorooctyl)thio)imidazolium-3-yl) propan-1-sulfonat | 160 |
| 3-((3-octyl-1-(1 H,1H,2H,2H-perfluorooctyl)imidazolium-2-yl)thio)propan-1-sulfonat | 2 |
| 1-Methyl-2-((1 H,1H,2H,2H-perfluorooctyl)thio)imidazolium-(±)-Kampfer-10-sulfonat | > 4000 |

Bei manchen Verbindungen bildete sich ein hochviskoses Gel aus, welches allerdings keinen sichtbaren Feststoff Rückstand aufwies.

Weitere Versuche zeigten, dass sich die erfindungsgemäßen Fluortenside auch in klebende Zusammensetzungen wie zum Beispiel in klebenden Hydrogelen einarbeiten lassen und nach wie vor eine Adhäsion auf verschiedenen Substraten wie Stahl oder menschlicher Haut erreicht wird.

### Beispiel 10:

### Bestimmung der Oberflächenspannung:

Die Bestimmung der statischen Oberflächenspannung für 3-(1H,1H,2H,2H-perfluorooctyl)- 2-((1H,1H,2H,2H-perfluorooctyl)thio)-1-methyl-1H-imidazolium chlorid in Wasser ergab die in der nachfolgenden Tabelle 6 angegebenen Werte für die Oberflächenspannung (OFS) in mN/m bei unterschiedlichen Konzentrationen c in Gew.%. Die Werte für OFS stellen den Mittelwert aus 10 Messungen dar. Die Standardabweichung S [mN/m] für diese Werte ist ebenfalls angegeben.

**Tabelle 6**

| **c (Gew.-%)** | **OFS (mN/m)** | **S [mN/m]** |
|---|---|---|
| 0,5000 | 14,5 | 0,04 |
| 0,2500 | 15,5 | 0,04 |
| 0,1000 | 15 | 0,1 |
| 0,0750 | 15,02 | 0,05 |
| 0,0500 | 15,05 | 0,04 |
| 0,0250 | 14,43 | 0,03 |
| 0,0100 | 16,29 | 0,04 |
| 0,0075 | 18,89 | 0,05 |
| 0,0050 | 21,29 | 0,05 |
| 0,0025 | 40,74 | 0,4 |

Die Messwerte zeigen, dass die Oberflächenspannung von Wasser bei der Zugabe des Tensids deutlich abnimmt, wobei das Tensid selbst bei sehr geringen Konzentrationen noch einen großen Effekt zeigt.

### Beispiel 11:

### Fluortensid für die Anwendung als Schiwachs:

95 g eines Paraffinwachses wurden bei 150°C geschmolzen und es wurden 5 g 1-octyl- 3-(1H,1H,2H,2H-perfluorooctyl)- 2-((1H,1H,2H,2H-perfluorooctyl)thio)- 1H-imidazoliumiodid zugegeben. Die Mischung wurde intensiv gerührt, bis eine homogene Schmelze erkennbar war. Die Schmelze wurde durch Abkühlen auf Raumtemperatur zum Erstarren gebracht.

Die so erhaltene Wachsmischung wurde mit einem Bügeleisen auf den Belag eines Langlaufskis aufgetragen. Die Gleiteigenschaften dieses Skis wurden mit den Gleiteigenschaften eines mit dem reinen Paraffinwachs behandelten Skis unter frühlingshaften Schneebedingungen verglichen. Der mit einem erfindungsgemäßen Wachs behandelte Ski zeigte deutlich bessere Gleiteigenschaften.

### Beispiel 12:

### Fluortensid für die Anwendung als Schaumbildner:

Die in der nachfolgenden Tabelle 7 gelisteten Komponenten wurden gemischt und die Mischung wurde bis zur Homogenität im Ultraschallbad geschüttelt.

**Tabelle 7**

| **Stoff** | **Menge** |
|---|---|
| 3-(1 H,1H,2H,2H-perfluorooctyl)-1-vinyl-1H-imidazoliumchlorid | 2 g |
| 2,2-Azobis(2-methylpropionamidin)-dihydrochlorid | 0,1 g |
| Wasser | 95,5 g |

Die erhaltene homogene Mischung wurde auf 65°C erhitzt, um eine Polymerisationsreaktion zu starten. Nach 6 h wurde auf Raumtemperatur gekühlt. Es wurde eine stabile Emulsion erhalten. Anschließend wurden 1,9 g 2-((1H,1H,2H,2H-perfluorooctyl)thio)-1-methyl-1 H-imidazoliumchlorid und 0,5 g 3-(1 H, 1H,2H,2H-perfluorooctyl)-2-((1 H, 1H,2H,2H-perfluorooctyl)thio)-1-methyl-1H-imidazoliumchlorid zugegeben und im Ultraschallbad wurden die beiden Komponenten vorsichtig gelöst.

Die so erhaltene Mischung zeichnete sich durch eine besonders schnelle und stabile Schaumbildung aus. Diese Eigenschaft ist beispielsweise bei einer Anwendung in Feuerlöschschäumen von Bedeutung.

## Patentansprüche

1. Zusammensetzung umfassend ein fluorhaltiges Tensid mit einer kationischen Gruppe, einer zwei- oder mehrwertigen schwefelhaltigen Gruppe und einer fluorierten Gruppe, sowie ferner umfassend ein zu der kationischen Gruppe des fluorhaltigen Tensids korrespondierendes Anion, **dadurch gekennzeichnet,**
**dass** es sich bei der kationischen Gruppe um eine N,N-disubstituierte Imidazoliumgruppe, eine N,N-disubstituierte Benzimidazoliumgruppe oder eine N,N-disubstituierte Benzimidazoliniumgruppe handelt, und dass es sich bei der fluorierten Gruppe um eine vollständig fluorierte Kohlenwasserstoffgruppe vom Typ -(CF₂)ₙ-CF₃ handelt, wobei n zwischen 2 und 9 liegt.

2. Zusammensetzung nach Anspruch 1, **dadurch gekennzeichnet, dass** n 4, 5 oder 6 ist.

3. Zusammensetzung nach einem der vorhergehenden Ansprüche, **dadurch gekennzeichnet, dass** es sich bei der schwefelhaltigen Gruppe um einen Thioether handelt.

4. Zusammensetzung nach einem der vorhergehenden Ansprüche, **dadurch gekennzeichnet, dass** das Anion ein Fluorid, Chlorid, Bromid, lodid, Arylsulfonat, Alkylsulfonat, Alkylsulfat, Sulfat, Arylphosphonat, Alkylphosphonat, Monoalkylphosphat, Dialkylphosphat, (Di)Hydrogenphosphat, Phosphat, Hexafluorophosphat, Hydrogencarbonat, Carbonat, Carbamat, Alkylcarbonat, Trifluoromethansulfonat, Bis(trifluormethansulfonyl)imid, Nonaflat oder Carboxylat umfasst.

5. Zusammensetzung nach einem der vorhergehenden Ansprüche, **dadurch gekennzeichnet, dass** das Anion nicht kovalent an die kationische Gruppe gebunden ist.

6. Zusammensetzung nach Anspruch 5, **dadurch gekennzeichnet, dass** das nicht kovalent an die kationische Gruppe gebundene Anion eine polymerisierbare Gruppe, vorzugsweise eine Vinyl-, (Meth)acryl- oder (Meth)acrylamidgruppe, und/oder eine fluorierte Gruppe umfasst.

7. Zusammensetzung nach einem der vorhergehenden Ansprüche, **dadurch gekennzeichnet, dass** das Anion kovalent an das fluorhaltige Tensid gebunden ist.

8. Zusammensetzung nach Anspruch 7, **dadurch gekennzeichnet, dass** das kovalent gebundene Anion mit einem zwischengelagerten Spacer an ein Stickstoffatom der kationischen Gruppe gebunden ist oder dass die schwefelhaltige Gruppe zwischen der kationischen Gruppe und dem kovalent gebundenen Anion angeordnet ist, wobei das Anion direkt oder mit einem zwischengelagerten Spacer an die schwefelhaltige Gruppe gebunden ist.

9. Zusammensetzung nach einem der vorhergehenden Ansprüche, dass die fluorierte Gruppe direkt oder mit einem zwischengelagerten Spacer an ein Stickstoffatom der kationischen Gruppe substituiert ist oder dass die schwefelhaltige Gruppe zwischen der kationischen Gruppe und der fluorierten Gruppe angeordnet ist, wobei die fluorierte Gruppe direkt oder mit einem zwischengelagerten Spacer an die schwefelhaltige Gruppe gebunden ist.

10. Zusammensetzung nach Anspruch 8 oder 9, **dadurch gekennzeichnet, dass** es sich bei dem Spacer um eine lineare oder verzweigte C1-C10, vorzugsweise C1-C5 und weiter vorzugsweise C2-C3 Alkylengruppe, insbesondere um eine Ethylengruppe handelt.

11. Zusammensetzung nach einem der vorhergehenden Ansprüche, **dadurch gekennzeichnet, dass** das fluorhaltige Tensid eine der folgenden Strukturen aufweist: 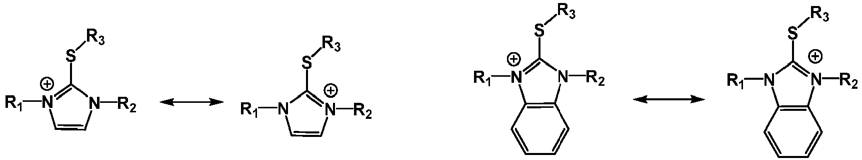 wobei einer der Reste R₁, R₂ oder R₃ eine (CH₂)₁₋₅(CF₂)₃₋₁₀CF₃ Gruppe darstellt, und die beiden weiteren der Reste unabhängig voneinander Wasserstoff oder eine lineare oder verzweigte C1-C10 Alkylgruppe darstellen, die gegebenenfalls eine vernetzbare Gruppe oder eine kovalent gebundene anionische Gruppe aufweisen kann.

12. Verwendung der Zusammensetzung nach einem der vorhergehenden Ansprüche als oberflächenaktives Mittel und vorzugsweise zur Verringerung der Oberflächenspannung, als Entschäumer, zur Einstellung optischer Eigenschaften oder zur Einstellung der Sauerstofflöslichkeit oder - permeabilität.

13. Verfahren zur Herstellung einer Zusammensetzung nach einem der Ansprüche 1 bis 11,
**dadurch gekennzeichnet,**
**dass** es den Schritt einer Umsetzung einer ungeladenen Verbindung mit einer N-alkylierten heterozyklischen und insbesondere heteroaromatischen Gruppe, einer divalenten schwefelhaltigen Gruppe und einer fluorierten Gruppe mit einer Säure umfasst.

14. Verfahren zur Herstellung einer Zusammensetzung nach einem der Ansprüche 1 bis 11,
**dadurch gekennzeichnet,**
**dass** das Verfahren eine Metathese zum Austausch des mit der kationischen Gruppe des fluorhaltigen Tensids korrespondierenden Anions umfasst.

## Claims

1. A composition comprising a surfactant containing fluorine and having a cationic group, a bivalent or polyvalent sulfurous group, and a fluorinated group and further comprising an anion corresponding to the cationic group of the surfactant containing fluorine,
**characterized in that**
the cationic group is an N,N-disubstituted imidazolium group, an N,N-disubstituted benzimidazolium group or an N,N-disubstituted benzimidazolinium group, and that the fluorinated group is a fully fluorinated hydrocarbon group of the type -(CF₂)ₙ-CF₃ where n is between 2 and 9.

2. A composition in accordance with claim 1, **characterized in that** n is 4, 5, or 6.

3. A composition in accordance with one of the preceding claims, **characterized in that** the sulfurous group is a thioether.

4. A composition in accordance with one of the preceding claims, **characterized in that** the anion comprises a fluoride, chloride, bromide, iodide, aryl sulfonate, alkyl sulfonate, alkyl sulfate, sulfate, aryl phosphonate, alkyl phosphonate, monoalkyl phosphate, dialkyl phosphate, (di)hydrogen phosphate, phosphate, hexafluorophosphate, hydrogen carbonate, carbonate, carbamate, alkyl carbonate, trifluoromethanesulfonate, bis(trifluoromethane sulfonyl)imide, nonaflate, or carboxylate.

5. A composition in accordance with one of the preceding claims, **characterized in that** the anion is non-covalently bonded to the cationic group.

6. A composition in accordance with claim 5, **characterized in that** the anion non-covalently bonded to the cationic group includes a polymerizable group, preferably a vinyl, (meth)acrylic or (meth)acrylamide group, and/or a fluorinated group.

7. A composition in accordance with one of the preceding claims, **characterized in that** the anion is covalently bonded to the surfactant containing fluorine.

8. A composition in accordance with claim 7, **characterized in that** the covalently bonded anion is bonded with an interposed space to a nitrogen atom of the cationic group; or **in that** the sulfurous group is arranged between the cationic group and the covalently bonded anion, with the anion being bonded to the sulfurous group directly or with an interposed spacer.

9. A composition in accordance with one of the preceding claims, **characterized in that** the fluorinated group is substituted at a nitrogen atom of the cationic group directly or with an interposed spacer; or **in that** the sulfurous group is arranged between the cationic group and the fluorinated group, with the fluorinated group being bonded to the sulfurous group directly or with an interposed spacer.

10. A composition in accordance with claim 8 or 9, **characterized in that** the spacer is a linear or branched C1-C10, preferably C1-C5, and further preferably C2-C3 alkylene group, in particular an ethylene group.

11. A composition in accordance with one of the preceding claims, **characterized in that** the surfactant containing fluorine has one of the following structures: 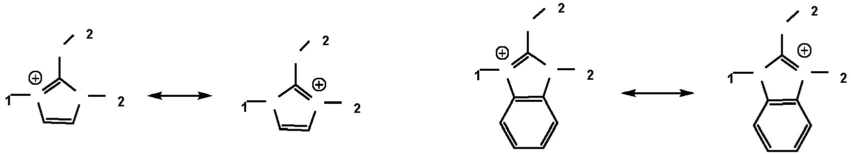 where one of the residues R₁, R₂ or R₃ represents a (CH₂)₁₋₅(CF₂)₃₋₁₀CF₃ group and the two further ones of the residues represent, independently of one another, hydrogen or a linear or branched C1-C10 alkyl group that can optionally have a crosslinkable group or a covalently bonded anionic group.

12. Use of the composition in accordance with one of the preceding claims as a surface-active agent, and preferably for reducing the surface tension, as a defoaming agent, for setting optical properties, or for setting the oxygen solubility or oxygen permeability.

13. A method of preparing a composition in accordance with one of the claims 1 to 11,
**characterized in that**
it comprises the step of a conversion of an uncharged compound having an N-alkylated heterocyclic and in particular heteroaromatic group, a divalent sulfurous group, and a fluorinated group with an acid.

14. A method of preparing a composition in accordance with one of the claims 1 to 11,
**characterized in that**
the method comprises a metathesis for exchanging the anion corresponding to the cationic group of the surfactant containing fluorine.

## Revendications

1. Composition comprenant un tensioactif fluoré avec un groupe cationique, un groupe sulfureux bivalent ou polyvalent, et un groupe fluoré, et comprenant en outre un anion correspondant au groupe cationique du tensioactif fluoré,
**dans laquelle,**
le groupe cationique est un groupe imidazolium N,N-disubstitué, un groupe benzimidazolium N,N-disubstitué ou un groupe benzimidazolinium N,N-distribué, dans laquelle le groupe fluoré est un groupe hydrocarboné entièrement fluoré de type -(CF₂)ₙ-CF₃, où n est entre 2 et 9.

2. Composition selon la revendication 1, dans laquelle n vaut 4, 5 ou 6.

3. Composition selon l'une des revendications précédentes, dans laquelle le groupe sulfureux est un thioéther.

4. Composition selon l'une des revendications précédentes, dans laquelle l'anion comprend un fluorure, un chlorure, un bromure, un iodure, un sulfonate d'aryle, un sulfonate d'alkyle, un sulfate d'alkyle, un sulfate, un phosphonate d'aryle, un phosphonate d'alkyle, un phosphate de monoalkyle, un phosphate de dialkyle, un (di)hydrogénophosphate, un phosphate, un hexafluorophosphate, un hydrogénocarbonate, un carbonate, un carbamate, un carbonate d'alkyle, un trifluorométhanesulfonate, un bis(trifluorométhanesulfonyle)imide, un nonaflate ou un carboxylate.

5. Composition selon l'une des revendications précédentes, dans laquelle l'anion n'est pas lié de manière covalente au group cationique.

6. Composition selon la revendication 5, dans laquelle l'anion qui n'est pas lié de manière covalente au groupe cationique comprend un groupe polymérisable de préférence, un groupe vinyle, (méth)acryl- ou (méth)acrylamide et / ou un groupe fluoré.

7. Composition selon l'une des revendications précédentes, dans laquelle l'anion est lié de manière covalente au tensioactif fluoré.

8. Composition selon la revendication 7, dans laquelle l'anion lié de manière covalente est lié à un atome d'azote du groupe cationique par l'intermédiaire d'un espaceur intercalé ou dans laquelle le groupe sulfureux est disposé entre le groupe cationique et l'anion lié de manière covalente, de sorte que l'anion soit lié directement ou avec un espaceur intercalé au groupe sulfureux.

9. Composition selon l'une des revendications précédentes, dans laquelle le groupement fluoré est substitué soit directement, soit par un espaceur intercalé sur un atome d'azote du groupe cationique, ou le groupe sulfureux est disposé entre le groupe cationique et le groupe fluoré, où le groupe fluoré est lié soit directement, soit via un espaceur intercalé sur le groupe sulfureux.

10. Composition selon la revendication 8 ou 9, dans laquelle l'espaceur est un groupe alkyle, en particulier un groupe éthyle, linéaire ou ramifié en C1-C10, de préférence en C1-C5 et plus préférentiellement en C2-C3.

11. Composition selon l'une des revendications précédentes, dans laquelle le tensioactif fluoré possède l'une des structures suivantes : 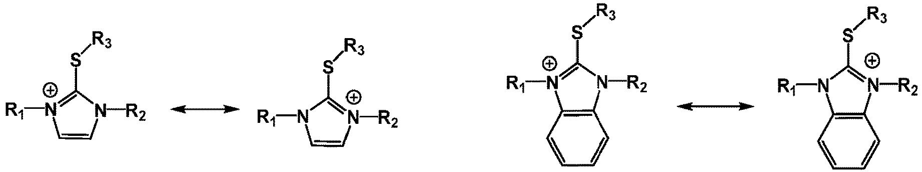 où l'un des résidus R₁, R₂ ou R₃ représente un groupe (CH₂)₁₋₅(CF2)₃₋₁₀CF₃ et les deux autres résidus représentent, indépendamment l'un de l'autre, hydrogène ou un groupe alkyle linéaire ou ramifié C1-C10 qui peut éventuellement posséder un groupe réticulable ou lié de manière covalente à l'anion.

12. Application de la composition selon l'une des revendications précédentes comme agent tensioactif, de préférence pour réduire la tension superficielle, comme antimousse, pour définir les propriétés optiques ou pour ajuster la solubilité ou la perméabilité de l'oxygène.

13. Procédé de préparation d'une composition selon l'une des revendications 1 à 11,
**dans lequel,** ledi
t procédé comprend l'étape de réaction d'un composé non chargé ayant un groupe hétérocyclique N-alkylé et en particulier hétéroaromatique, un groupe sulfureux divalent, et un groupe fluoré avec un acide.

14. Procédé de préparation d'une composition selon l'une des revendications 1 à 11,
**dans lequel,**
ledit procédé comprend une métathèse pour échanger l'anion correspondant au groupe cationique du tensioactif fluoré.
